# EUROPEAN PATENT APPLICATION

(11) **EP 4 755 445 A1**
(43) Date of publication of application: **10.06.2026**
(21) Application number: 24307059.6
(22) Date of filing: 09.12.2024
(51) Int. Cl.: A61N 5/06

(54) **SYSTEM, EYEWEAR AND METHOD FOR IMPROVING FUTURE VISUAL PERFORMANCE OR FUTURE COGNITIVE PERFORMANCE**

(71) Applicant: Essilor International, 94220 Charenton-Le-Pont (FR); SORBONNE UNIVERSITE, 75006 Paris (FR)
(72) Inventor: DUBAIL, Marie, 94410 SAINT-MAURICE (FR); LASSALLE, Astrid, 94100 SAINT-MAUR-DES-FOSSÉS (FR); SIDIBE, Coraline, 93200 SAINT-DENIS (FR)
(74) Representative: Plasseraud IP

(57) **Abstract**

System, eyewear and method for improving future visual performance or future cognitive performance of the individual. The system comprises a device for irradiating light toward an eye of the individual. The system is configured for:
• determining, at a first time, first visual performance or first cognitive performance of the individual,
• determining values of parameters of a first light to be irradiated toward an eye of the individual according to the first visual performance or the first cognitive performance of the individual,
• irradiating, between the first time and a second time, the first light toward the eye of the individual,
• determining, at the second time, second visual performance or second cognitive performance of the individual,
• irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

## Description

### Technical field

This disclosure relates to systems and methods for improving future visual performance or future cognitive performance of an individual.

### Background information and prior art

The visual performance of an individual may be defined as their ability to detect, perceive or discriminate a visual stimulus. Visual performance may be represented by a subjective or objective measure of a parameter of a visual function of an individual. The visual function may include visual acuity, static or dynamic contrast sensitivity, perimetry, or oculomotor performance.

The cognitive performance of the individual may be defined as their ability to perform cognitive tasks such as attention, inhibition, learning, and memory (short-term memory, long-term memory or working memory). The cognitive performance may be assessed through the execution of psychophysical tests. More precisely the accuracy number of right answer, reaction time, thresholds (acquisition, maintenance, retrieval time) of the individual during the execution of the psychophysical tests may be used to evaluate their cognitive performance.

There is a need for systems and methods for improving future visual performance or future cognitive performance of an individual.

### Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a system for improving future visual performance or future cognitive performance of the individual. The system comprises a device for irradiating light toward an eye of the individual. The system is configured for:
- determining, at a first time, first visual performance or first cognitive performance of the individual,
- determining values of parameters of a first light to be irradiated toward an eye of the individual according to the first visual performance or the first cognitive performance of the individual,
- irradiating, between the first time and a second time, the first light toward the eye of the individual,
- determining, at the second time, second visual performance or second cognitive performance of the individual,
- irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

This system is using light therapy to improve the future visual performance and the future cognitive performance of the individual. Furthermore, the light irradiating the eye of the individual is adapted specifically to the individual, more precisely to current visual performance or current cognitive performance of the individual.

The benefits for the individual may be, regarding cognitive performance, an increase of the attention, an increase of the working memory and regarding visual performance an increase of the fixation stability, a decrease (or improvement) of the reaction time before ocular movements and a higher precision of the ocular movements. The individual may also more easily fall asleep at night and be more relaxed.

Furthermore, this system takes into account the evolution of both visual and cognitive performance to determine the values of the parameters of the irradiated light.
Another aspect of this disclosure is an eyewear for improving future visual performance or future cognitive performance of an individual. The eyewear is configured for:
- determining, at a first time, first visual performance or first cognitive performance of the individual,
- determining, values of parameters of a first light to be irradiated toward an eye of the individual according to the first visual performance or the first cognitive performance of the individual,
- irradiating, between the first time and a second time, the first light toward the eye of the individual,
- determining, at the second time, second visual performance or second cognitive performance of the individual,
- irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

Another aspect of this disclosure is a method for improving future visual performance or future cognitive performance of an individual. The method comprises:
- determining, at a first time, first visual performance or first cognitive performance of the individual,
- determining, values of parameters of a first light to be irradiated toward an eye of the individual according to the first visual performance or the first cognitive performance of the individual,
- irradiating, between the first time and a second time, the first light toward the eye of the individual,
- determining, at the second time, second visual performance or second cognitive performance of the individual,
- irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

Another aspect of this disclosure is a computer program comprising instructions which, when the computer program is executed by a computer, cause the computer to carry out a method for improving future visual performance or future cognitive performance of an individual. The method comprises:
- determining, at a first time, first visual performance or first cognitive performance of the individual,
- determining, values of parameters of a first light to be irradiated toward an eye of the individual according to the first visual performance or the first cognitive performance of the individual,
- irradiating, between the first time and a second time, the first light toward the eye of the individual,
- determining, at the second time, second visual performance or second cognitive performance of the individual,
- irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

Another aspect of this disclosure is a non-transitory program storage device, readable by a computer, tangibly embodying a program of instructions executable by the computer to perform a method for improving future visual performance or future cognitive performance of an individual. The method comprises:
- determining, at a first time, first visual performance or first cognitive performance of the individual,
- determining, values of parameters of a first light to be irradiated toward an eye of the individual according to the first visual performance or the first cognitive performance of the individual,
- irradiating, between the first time and a second time, the first light toward the eye of the individual,
- determining, at the second time, second visual performance or second cognitive performance of the individual,
- irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

### Description of the drawings

For a more complete understanding of the description provided herein and the advantages thereof, reference is now made to the brief descriptions below, taken in connection with the accompanying drawings and detailed description, wherein like reference numerals represent like parts.
Figure 1 represents a system for improving the future visual performance or the future cognitive performance of the individual.
Figure 2 represents eyewear.
Figure 3 represents a removable clip attachable to the eyewear.
Figure 4 represents the method for improving the future visual performance or the future cognitive performance of the individual.

### Detailed description of embodiments

The detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### Description of the system for improving the future visual performance or the future cognitive performance of the individual

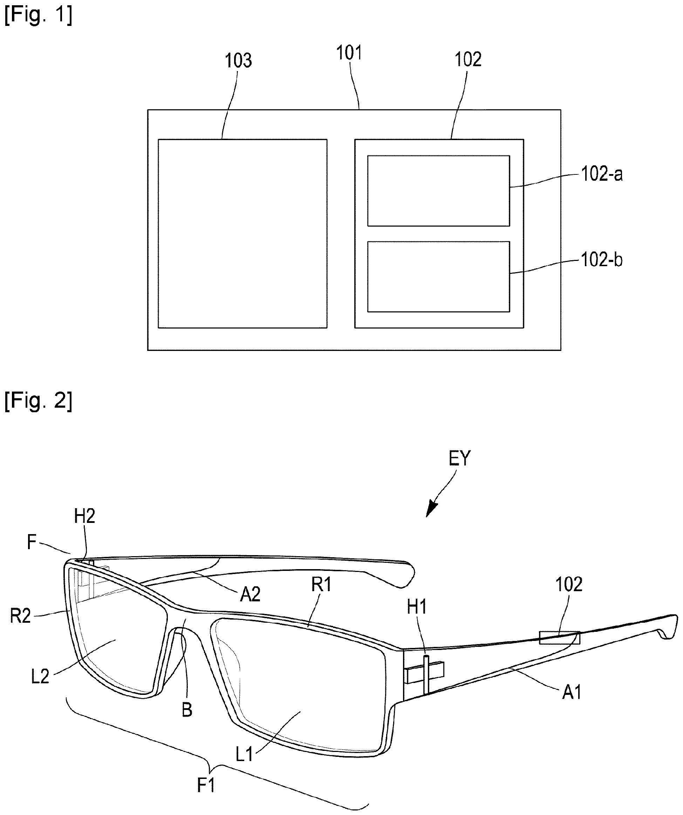

Figure 1 represents a system 101 for improving the future visual performance or the future cognitive performance of the individual. The system 101 may comprise a calculation module 102 and a device 103 for irradiating light toward an eye of the individual.

**The** system 101, and more precisely the calculation module 102, may be configured for determining values of parameters of the light according to visual performance or cognitive performance of the individual.

The calculation module 102 may also be configured to conduct tests to the individual and for determining values of the visual performance or the cognitive performance of the individual based on results of the tests.

**The** device 103 may comprise a lighting module. The lighting module may comprise a plurality of LEDs for example between 1 and 7 LEDs. One may have a minimum of 3 LEDs, preferably 6. One may place diffusers in front of the LEDs to avoid having directional light.

**The** lighting module may comprise at least one pass-band filter, the at least one pass-band filter being placed in front of the at least one LED. This allow the light irradiated by the at least one LED to be filtered before reaching the individual.

The device 103 may comprise a passive optical element configured to modify environmental light reaching the eye of the individual. The passive optical element may be a filter.

The device 103 may comprise an active optical element configured to modify environmental light reaching the eye of the individual.

By an active optical element, one means an optical element in which a value of a parameter of a functionality of the optical device can be modified on-demand. The value of the parameter may be modified by applying a signal, for example an electrical signal to terminals of the active optical device. The functionality may be one of the following:
- a transmittance level of the active optical element,
- a tint of the active optical element,
- a reflectance level of the active optical element and
- a parameter of a polarization of the active optical device, for example a polarization angle of the active optical device.

The device 103 may be a head-mountable device.

By "head-mountable device", one means a device designed to be mounted on the head of the individual. The head-mountable device may perform several functions, some of which are related to the eyes of the individual.

Examples of head-mountable devices include eyewear, helmets, and clips that can be attached to a cap or eyewear, or a mask.

The head-mountable device may be a headset or may also comprise at least one audio speaker.

By "eyewear" one means a device designed to be worn in front of one or both eyes of the individual. The eyewear may have a function. The function may be a vision improvement function or a vision correction function. The function may be the protection of the eyes of the individual. The eyewear may be eyeglasses, augmented reality eyeglasses or virtual reality eyeglasses.

The figure 2 represents the eyeglasses EY, which are an example of the device 103. The eyeglasses EY comprise two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. The eyeglasses EY can be prescription eyeglasses for which the refraction has been adapted to the individual by an eye care professional and eyewear without refraction for example sunglasses.

The device 103 for irradiating the light may be home tunable lights on which the values of the parameters of the light irradiated by the home tunable lights may be modified, for example may be modified by the calculation module 102.

The calculation module 102 may comprise a memory 102-a and a processor 102-b.

The calculation module may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

The device 103 may comprise the calculation module 102.

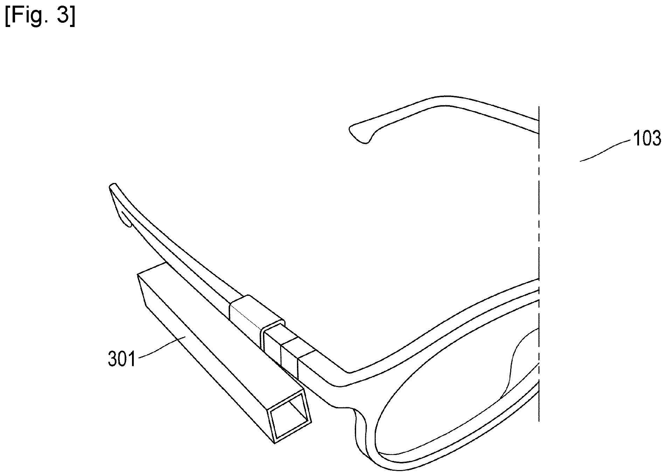

As represented in the figure 3, the eyeglasses EY may integrate the calculation module 102. The calculation module 102 may be located in one or both of the arms A1 and A2 or in the front face F1.

In other embodiments, represented figure 3, a removable clip 301 may be attached to the eyeglasses EY. This removable clip 301 may comprise the calculation module 102.

In other embodiments the calculation module 102 may be integrated in a smartphone or a watch, more precisely a wristwatch.

Wristwatches are designed to be worn around the wrist, attached by a watch strap or other type of bracelet, including metal bands, leather straps, or any other kind of bracelet.

The watch may also be a smartwatch that is a wearable computer in the form of a watch. Modern smartwatches provide generally a local touchscreen interface for daily use, while an associated smartphone application and may comprise sensors used to determine physiological parameters of the individual. The calculation module 102 may be included in the smartwatch and may be shared with other functions of the smartwatch.

**The** calculation module 102 may be a computer or may be a virtual machine located on a cloud network, or a server not co-located with the individual.

**The** system 101 may also be synchronized with the individual alarm clock to let the system 101 start the irradiation of light just prior the wake-up of the individual.

When the device 103 is the head-mountable device, the device 103 may be configured to irradiate the light preferably when the individual is wearing the head-mountable device.

The device 103 may also comprise a light sensor adapted to determine values of the parameters of the light reaching the eye of the individual.

### Description of the method for improving the future visual performance or the future cognitive performance of the individual

To improve the future visual performance or the future cognitive performance of the individual, the memory 102-a may store a computer program comprising instructions which, when the program is executed by the processor 102-b, cause the calculation module 102 to carry out a method, for example a computer implemented method, for improving the future visual performance or the future cognitive performance of the individual.

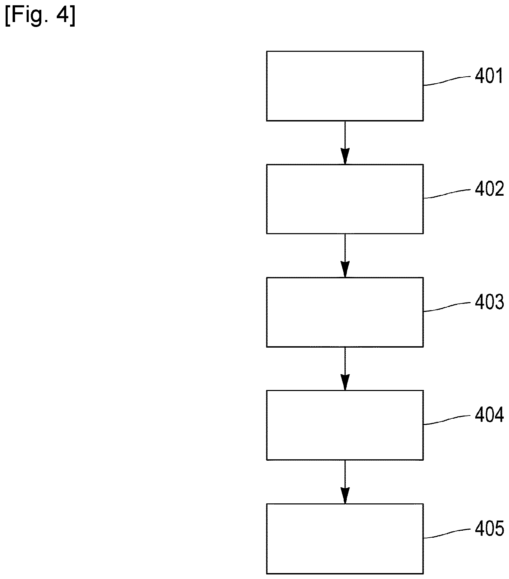

The method, as presented in figure 4, comprises:
- a step of determining 401, at a first time, first visual performance or first cognitive performance of the individual,
- a step of determining 402, at the first time, values of parameters of a first light to be irradiated toward an eye of the individual according to the first visual performance or the first cognitive performance of the individual,
- a step of irradiating 403, between the first time and a second time, the first light toward the eye of the individual,
- a step of determining 404, at the second time, second visual performance or second cognitive performance of the individual,
- a step of irradiating 405, after the second time, a second light toward the eye of the individual.
When the second visual performance is better than the first visual performance or the second cognitive performance is better than the first cognitive performance, a light dose irradiated by the second light may be greater than a light dose irradiated by the first light.

The light dose irradiated by a light (for example the first light or the second light), may be characterised by a combination of a duration of the irradiation of the light and an irradiance level of the light.

Therefore, the expression 'the light dose irradiated by the second light may be greater than the light dose irradiated by the first light' may indicate that the duration of irradiation by the second light is longer than that of the first light, that the irradiance level of the second light is higher than that of the first light, or both. It may also indicate that the system 101, more precisely the device 103, is configured to irradiate the second light during a longer duration of irradiation than the first light, to irradiate the second light with an irradiance level higher than the one of the first one, or both.

Therefore, the expression 'the light dose irradiated by the second light may be smaller than the light dose irradiated by the first light' may indicate that the duration of irradiation by the second light is shorter than that of the first light, that the irradiance level of the second light is lower than that of the first light, or both. It may also indicate that the system 101, more precisely the device 103, is configured to irradiate the second light during a shorter duration of irradiation than the first light, to irradiate the second light with an irradiance level smaller than the one of the first one, or both.

The first time may be prior to the second time.

The second time may be the current time. The first time may be a previous time.

The first visual performance may be previous visual performance.

The first cognitive performance may be previous cognitive performance.

The second visual performance may be previous visual performance.

The second cognitive performance may be previous cognitive performance.

The steps of determining (401, 402 and 404) may be realized the calculation module 102.

The steps of irradiating the light (first or second) may be realized by the device 103, more precisely by the lighting module of the device 103. In this case, the calculation module 102 will instruct the device 103, more precisely by the lighting module of the device 103, to irradiated the light.

When the device 103 is an eyewear comprising the lighting module and the calculation module 102, the steps of determining (401, 402 and 404) may be realized by the calculation module 102 and the steps of irradiating the light (first or second) may be realized by the lighting module. The lighting module may be instructed, by the calculation module 102, to realize the steps of irradiating the light (first or second).

In the method of figure 4, when the second visual performance is almost equal to the first visual performance or the second cognitive performance is almost equal to the first cognitive performance, the light dose irradiated by the second light may be smaller than the light dose irradiated by the first light.

When the light dose irradiated by the second light is smaller than the light dose irradiated by the first light, and the second visual performance is worse than the first visual performance or the second cognitive performance is worse than the first cognitive performance, one may irradiate the individual with a third light. A light dose irradiated by the third light may be greater than the light dose irradiated by the second light.

A daily, weekly or monthly duration of irradiation may be set initially. For example, the daily duration of irradiation may be set to 10 minutes.

The irradiated light may be composed of a plurality of wavelengths (preferably 6).

Example of lights are:
- Amber light to Re-Activate ipRGC (intrinsically photosensitive retinal ganglion cells),
- High level of melanopsin composed of 4 wavelengths to stimulate ipRGC with a white achromatic light to improve cognitive and visual functions, the ratio between the short and the high wavelengths may change depending on an age of the individual,
- Low level of melanopsin composed of 3 wavelengths to relax and favor sleep.

The light irradiated during the step 403 or 405 of irradiating light toward the eye of the individual may have a peak around 480nm. An irradiance level of the peak may be more than 1.10 W/m².The irradiance level, expressed in W/m², is determined at the entry of the eye. The irradiance level corresponds to the light entering the eye.

The light irradiated during the step 403 or 405 of irradiating light toward the eye of the individual may be a white polychromatic light. An irradiance level of the white polychromatic light may be more than 1.10 W/m², for example the irradiance level of the white polychromatic light may be of about 2.17 W/m².

The white polychromatic light may also be defined by :
- Chromaticity coordinates in CIE chromaticity diagram : 0.2<y<0.4 and 0.2<x<0.55
- Chromaticity coordinates close to black body locus curve : euclidian distance < 0.14
- melanopic irradiance level superior to 0.9 W/m² as defined by CIE norm CIE S 026.

The irradiance level corresponds to the irradiance of the whole bandwidths of the light. Melanopic irradiance is calculated over the action spectrum of the ipRGC photoreceptor. ipRGC means intrinsically photosensitive Retinal Ganglion Cells. They are the cells containing melanopsin.

The light irradiated during the step 403 or 405 of irradiating light toward the eye of the individual may be a bichromatic light. The bichromatic light may have a first peak around 480nm and a second peak in a range from 590 nm to 620nm, an irradiance level of the first peak may be more than 1.10 W/m2 and an irradiance level of the second peak may be more than 1.10 W/m2.

The light irradiated during the step 403 or 405 of irradiating light toward the eye of the individual may be continuous.

The light irradiated during the step 403 of 405 of irradiating light toward the eye of the individual may flicker, preferably with a frequency of less than 60Hz.

The values, presented in the previous sections, of the parameters of the irradiated light are particularly adapted to elderly people. More precisely they are adapted to their crystalline lens transmission that is 1.10 time lower than the crystalline lens transmission of young people.

For a light with a peak at 480nm, the irradiance level of 1.10 W/m² corresponds to 150 Lux, the irradiance level of 2.30W/m² corresponds to 272 Lux.

The light irradiated during the step 403 of 405 of irradiating light toward the eye of the individual may have a wavelength between 590nm and 680nm. The irradiance level may be of less than 2.30 W/m².

The method of figure 4 may also comprise an initialization step. During this initialization step values of different parameters of the individual are obtained via for example a questionnaire. The parameters may be an age of the individual, whether or not the individual had cataract surgery, weather the individual has short-term goals or long-term goals, an amount of time the individual may spend, for example daily, weekly or monthly, using the system 101. The values of the parameters of the light irradiated toward the individual may depend on the values of these parameters.

During the initialization step, the luminance may be increased from 1lux to 1000lux until the individual precise that he reaches a light comfort threshold in order to remain in the comfort zone. A minimum of 150 lux in the shortest wavelengths may be used to reach increases performance during morning.

The age of the individual may be considered when determining the value of the parameters of the light. More precisely, one may vary gradually, following the age of the individual, a minimal irradiance level of the light and the wavelength of the peak of the light. To realize this variation, one may take into account the theoretical curve of the light transmission level of the crystallin of the eye of the individual. The wavelengths of the peaks may be shifted to longer wavelengths with age and the irradiance level may be increased with age. These allow the mitigation of the reduction of the mean size of the pupil and the decrease of the transmission level of the crystallin. When the individual underwent cataract surgery, the values of the parameters may be adjusted accordingly.

The method of figure 4 may also comprise a step of recommendation of a lens for the individual. This recommendation may depend on the current visual performance or the current cognitive performance.

The method of figure 4 may also comprise a step of determining a diameter of a pupil of the individual and the irradiance level of the light may be adapted accordingly to keep the retinal illuminance by the light constant.

The method of figure 4 may also comprise a step of adapting the irradiated light according to the value of the parameters of the light reaching the eye of the individual. This allows to considering the natural light reaching the eye of the individual.

The irradiated light may be also adapted to a program selected by the individual or a desired effect selected by the individual. The wavelength of the peaks of the light may be adjusted according to the program or the desired effect selected.

If the individual chooses the "performance" program, a light with more peaks and with the peaks having shorter wavelengths will be irradiated. The goal will be to provide a high melanopsin exposure and a high melanopic stimulation with a combination of natural exposure and additional exposure. On the contrary, if a relaxing mode is chosen, light with longer wavelengths and lower irradiance level will be transmitted, preferably to provide a low melanopic stimulation.

If the individual is using a "regular activities" program, the light will be irradiated without interaction of the individual.

If the individual is using a "training" program, the system 101 may propose, to the individual, a cognitive and visual training program while irradiating the light.

If the individual is using a "tasks" program, the system 101 may propose, to the individual, cognitive tasks or visual tasks. These tasks may test working memory, attention, inhibition but also visual performance.

If the individual is using a "relaxation" program, the system 101 may irradiated a light adapted to relaxation.

The method of figure 4 may also determine the values of the parameters of the irradiated light according to a time of day. The irradiated light may have a higher irradiance level and with peaks having shorter wavelengths in the morning than in the evening. This allows the individual to receive a high dose of melanopic stimulation in the morning and a low dose of melanopic stimulation in the evening.

## Claims

1. System (101) for improving future visual performance or future cognitive performance of an individual,
the system (101) comprising a device (103) for irradiating light toward an eye of the individual, the system (101) being configured for:
• determining, at a first time, first visual performance or first cognitive performance of the individual,
• determining values of parameters of a first light to be irradiated toward an eye of the individual according to the first visual performance or the first cognitive performance of the individual,
• irradiating, between the first time and a second time, the first light toward the eye of the individual,
• determining, at the second time, second visual performance or second cognitive performance of the individual,
• irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

2. The system (101) according to the claim 1,
when the second visual performance is almost equal to the first visual performance or the second cognitive performance is almost equal to the first cognitive performance, the light dose irradiated by the second light being smaller than the light dose irradiated by the first light.

3. The system (101) according to the claim 1 or 2,
the device (103) comprising at least one LED for irradiating the light.

4. The system (101) according to the claim 3,
the device (103) comprising at least one diffuser, the at least one diffuser being placed in front of the at least one LED.

5. The system (101) according to the claim 3,
the device (103) comprising at least one pass-band filter, the at least one pass-band filter being placed in front of the at least one LED.

6. The system (101) according to the claim any one of the claims 1 to 5,
the device (103) may comprise an active optical element configured to modify environmental light reaching the eye of the individual.

7. The system (101) according to any one of the claims 1 to 6,
the irradiated light having a peak around 480nm, an irradiance level of the peak being more than 1.10 W/m².

8. The system (101) according to any one of the claims 1 to 6,
the irradiated light being a white polychromatic light, an irradiance level of the white polychromatic light being more than 1.10 W/m².

9. The system (101) according to any one of the claims 1 to 6,
the irradiated light being a bichromatic light, the bichromatic light having a first peak around 480nm and a second peak in a range from 590 nm to 620nm, an irradiance level of the first peak being more than 1.10 W/m² and an irradiance level of the second peak being more than 1.10 W/m².

10. The system (101) according to any one of the claims 1 to 6,
a range of wavelength of the irradiated light being between 590nm and 680nm, an irradiance level of the light being less than 2.30 W/m².

11. The system (101) according to any one of the claims 1 to 10,
the device (103) being a head mountable device for example eyewear.

12. The system (101) according to any one of the claims 1 to 11,
the system (101) comprising a control module (102) for determining the values of the parameters of the light according to the current visual performance or the current cognitive performance of the individual.

13. Eyewear for improving future visual performance or future cognitive performance of an individual,
the eyewear being configured for:
• determining, at a first time, first visual performance or first cognitive performance of the individual,
• determining, values of parameters of a first light to be irradiated toward an eye of the individual according to the first visual performance or the first cognitive performance of the individual,
• irradiating, between the first time and a second time, the first light toward the eye of the individual,
• determining, at the second time, second visual performance or second cognitive performance of the individual,
• irradiating, after the second time, a second light toward the eye of the individual,
when the second visual performance is better than the first visual performance or the second cognitive performance is better than the first cognitive performance, a light dose irradiated by the second light being greater than a light dose irradiated by the first light.

14. Method for improving future visual performance or future cognitive performance of an individual,
the method comprising:
• determining (401), at a first time, first visual performance or first cognitive performance of the individual,
• determining (402), values of parameters of a first light to be irradiated toward an eye of the individual according to first visual performance or first cognitive performance of the individual,
• irradiating (403), between the first time and a second time, the first light toward the eye of the individual,
• determining (404), at the second time, second visual performance or second cognitive performance of the individual,
• irradiating (405), after the second time, a second light toward the eye of the individual,
when the second visual performance is better than the first visual performance or the second cognitive performance is better than the first cognitive performance, a light dose irradiated by the second light being greater than a light dose irradiated by the first light.
